# EUROPEAN PATENT SPECIFICATION

(11) **EP 1 574 172 B1**
(45) Date of publication and mention of the grant of the patent: **24.10.2007**
(21) Application number: 05251367.8
(22) Date of filing: 08.03.2005
(51) Int. Cl.: A61B 17/15, A61B 17/17

(54) **Navigated orthopaedic guide**
Navigierte orthopädische Führungseinrichtung
Guide orthopédique navigué

(30) Priority: 08.03.2004 US 795830
(43) Date of publication of application: 14.09.2005
(73) Proprietor: Zimmer Technology, Inc., Warsaw IN 46580 (US)
(72) Inventor: Grimm, James E., Winona Lake, Indiana 46590 (US); McGinley, Shawn E., Fort Wayne, Indiana 46814 (US)
(74) Representative: Mays, Julie

(56) References cited:
- EP-A- 1 302 167
- WO-A-97/29710
- DE-A1- 10 207 035
- US-A1- 2002 068 942
- US-A1- 2002 198 531
- US-A1- 2003 220 689

## Description

### BACKGROUND

The present invention relates to surgical components used in conjunction with a surgical navigation system. In particular, the present invention relates to a navigated instrument for guiding subsequent components during an orthopaedic surgical procedure. EP-A-1302167 defines the preamble of claim 1.

Many surgical procedures are now performed with surgical navigation systems in which sensors detect tracking elements attached in known relationship to an object in the surgical suite such as a surgical instrument, implant, or patient body part. The sensor information is fed to a computer that then triangulates the three dimensional position of the tracking elements within the surgical navigation system coordinate system. Thus, the computer can resolve the position and orientation of the object and display the position and orientation for surgeon guidance. For example, the position and orientation can be shown superimposed on an image of the patient's anatomy obtained via X-ray, CT scan, ultrasound, or other imaging technology.

However, most orthopaedic surgical procedures are performed using conventional instruments in which the various components of the surgery are aligned mechanically by the surgeon by visualizing and/or palpating anatomic landmarks. During these procedures, orthopaedic components in the form of instruments to prepare a bone, provisional components to verify sizing, implant components and/or other suitable components are placed in a surgical site. These components often have position and orientation requirements for them to operate properly. For example, a bone cutting guide must be aligned on the bone in the proper orientation to guide a cutter to produce a cut surface in a desire location.

### SUMMARY

The present invention provides a navigated orthopaedic guide as defined in claim 1.

In one aspect of the invention, a navigated orthopaedic guide is provided for use with a surgical navigation system during an orthopaedic surgical procedure to establish a datum relative to a surgical site. The datum is able to be engaged by a subsequent surgical component to guide placement of the subsequent surgical component. The orthopaedic guide includes a body, means for being tracked by the surgical navigation system to position the orthopaedic guide at a desired position relative to the surgical site, and means for establishing a datum at a desired position relative to the surgical site.

In another aspect of the invention, a surgical system is provided for use during an orthopaedic surgical procedure at a surgical site of a patient's body. The system includes a surgical navigation system, an orthopaedic guide including means for being tracked by the surgical navigation system, and a surgical component. The orthopaedic guide includes means for establishing a datum at a desired position relative to the surgical site. The surgical component includes means for engaging the datum positioned by the orthopaedic guide to locate the surgical component at a desired position relative to the surgical site.

### BRIEF DESCRIPTION OF THE DRAWINGS

Various illustrative examples of the present invention will be discussed with reference to the appended drawings. These drawings depict only illustrative examples of the invention and are not to be considered limiting of its scope.
FIG. 1 is a perspective view of an illustrative navigated orthopaedic guide according to the present invention in use to establish a datum relative to a bone;
FIG. 2 is a perspective view of the bone of FIG. 1 showing the datum established with the navigated orthopaedic guide of FIG. 1;
FIG. 3 is a perspective view showing a surgical component positioned using the datum of FIG. 2;
FIG. 4 is a perspective view of an illustrative alternative arrangement for the orthopaedic guide of FIG. 1;
FIG. 5 is an exploded perspective view of an illustrative alternative arrangement for the orthopaedic guide of FIG. 1 having an adjustment mechanism;
FIG. 6 is a perspective view of the orthopaedic guide of FIG. 5 in use to establish a datum relative to a bone;
FIG. 7 is a perspective view showing a surgical component positioned using the datum of FIG. 6;
FIG. 8 is an exploded perspective view of an illustrative alternative arrangement for the orthopaedic guide of FIG. 1 having an adjustment mechanism;
FIG. 9 is a perspective view of the orthopaedic guide of FIG. 8 in use to establish a datum relative to a bone;
FIG. 10 is a perspective view of the orthopaedic guide of FIG. 8 in use to establish a datum relative to a bone;
FIG. 11 is a cross sectional view taken along line 11-11 of FIG. 10 with the bone omitted for clarity.

### DESCRIPTION OF THE ILLUSTRATED EMBODIMENTS

Embodiments of a navigated orthopaedic guide may be configured to guide a variety of surgical components. For example, a navigated orthopaedic guide may be used to establish a datum relative to a bone such as one or more pins, screws, bars, fins, rails, dovetails, planar surfaces, holes, slots, notches, and/or any other suitable datum in or on a bone. The datum may be used to reference the position and/or orientation of a subsequent surgical component including cutting instruments, reaming instruments, templates, drill guides, provisional implants, implants, and/or other components for any suitable surgical site. Examples of surgical sites include hip joints, knee joints, vertebral joints, shoulder joints, elbow joints, ankle joints, digital joints of the hand and feet, fracture sites, tumor sites, and/or other suitable orthopaedic surgical sites. The orthopaedic guide of the present invention may be used to establish datums that may be referenced by components that are not otherwise usable with a surgical navigation system. Thus, the orthopaedic guide may be used to provide the benefits of three dimensional surgical navigation technology while using existing non-navigated components. The orthopaedic guide may be configured to establish a separate intermediate datum or it may serve as the datum itself to engage and guide a subsequent surgical component directly. A guide that serves directly as the datum may include one or more pins, screws, bars, fins, rails, dovetails, planar surfaces, holes, slots, notches, and/or other feature that directly engages the subsequent component to guide it relative to a surgical site. For example, the orthopaedic guide may include a slot to receive and guide a cutter to produce a cut surface on a bone.

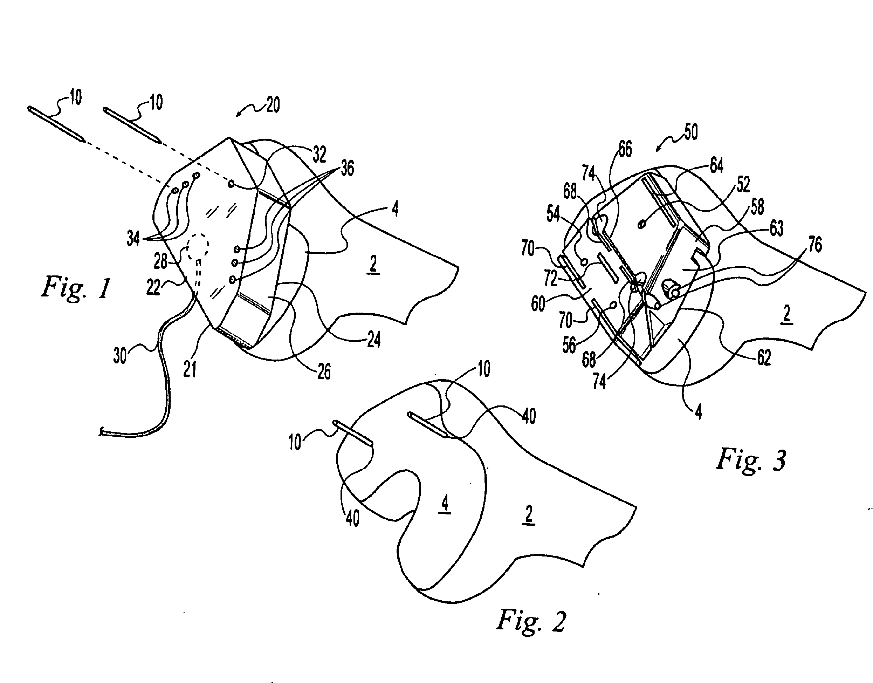

FIGS. 1-3 depict an illustrative navigated orthopaedic guide 20 configured to guide the placement of datum pins 10 on which a femoral cut guide 50 is positioned to guide the cutting of a femur 2 to receive a femoral component in knee replacement surgery. The guide 20 includes a body 21 having a front surface 22, a back surface 24 opposite the front surface 22, and a circumferential side wall 26 extending from the front surface 22 to the back surface 24. In the illustrative example, the orthopaedic guide 20 includes a tracking element in the form of an electromagnetic coil 28 embedded in the body 21 between the front and back surfaces 22, 24 and within the perimeter of the side wall 26. The coil 28 includes a lead 30 extending from the coil 28 and out of the body 21 to connect to the surgical navigation system for transmitting electrical signals between the surgical navigation system and the coil 28. When the coil 28 is place within an electromagnetic field, it generates an electrical charge that is transmitted to the surgical navigation system such that the three dimensional position and orientation of the coil 28, and thus the orthopaedic guide 20, can be related to a surgical navigation coordinate system. For example, the surgical navigation system may include multiple sensors at known locations that receive signals from the coil 28 and feed the information to a computer. The computer may then triangulate the three dimensional position of the coil within the surgical navigation coordinate system. The surgical navigation system may then determine the position and orientation of the orthopaedic guide 20 by detecting the position and orientation of the coil 28 and resolving the position and orientation of the orthopaedic guide 20 from the known relationship between the coil 28 and the orthopaedic guide 20.

While the illustrative example depicts an active electromagnetic tracking element, the tracking element may be detectable electromagnetically, acoustically, by imaging, or by other suitable detection means. Furthermore, the tracking element may be active or passive. Examples of active tracking elements may include electromagnetic field emitters in an electromagnetic system (such as the illustrative coil 28), light emitting diodes in an imaging system, and ultrasonic emitters in an acoustic system, among others. Examples of passive tracking elements may include elements with reflective surfaces. For example, reflective spheres or discs may be attached to the orthopaedic guide and detected by an imaging system.

The orthopaedic guide 20 includes means for establishing a datum on or in a bone to guide subsequent components. In the illustrative guide 20, holes 32, 34, 36 extend through the orthopaedic guide 20 from the front surface 22 to the back surface 24. The holes may guide the placement of pins 10, screws, or other datums. For example, a drill bit may be guided along one or more of the holes 32, 34, 36 to create a hole 40 (FIG. 2) in the underlying bone 2. A pin 10 may then be inserted into the hole in the bone 2. Alternatively, a self-drilling pin may be used. Alternatively, the pin 10 may be omitted and the hole 40 formed in the bone 2 may itself serve as a datum. Alternatively, the orthopaedic guide 20 may include a notch, slot, guide surface, or other feature to guide forming a notch, slot, or other datum in the bone 2. Alternatively, the orthopaedic guide 20 may include a slot, notch, guide surface, or other feature to guide placing a bar, rail, or other datum in or on the bone 2.
Once the datum has been positioned on the bone 2, a surgical component may be referenced to the datum to correctly position the surgical component. For example, in FIG. 3, a femoral cut guide 50 includes holes 52, 54, and 56 for receiving datum pins 10 set using the orthopaedic guide 20. Alternatively, the surgical component may include protrusions for engaging holes 40 formed using the orthopaedic guide 20, or other features for engaging other types of datums positioned using the orthopaedic guide 20. The femoral cut guide 50 includes a body 58 having a front surface 60, a back surface 62, and a circumferential side wall 63 extending from the front surface 60 to the back surface 62. The datum receiving holes 52, 54, 56 extend from the front surface 60 to the back surface 62. A plurality of slots 64, 66, 68, 70, 72 are formed through the cut guide 50 from the front surface 60 to the back surface 62 to guide a cutter to shape the end of the femur 2 to receive a femoral knee implant. For example, a posterior cut slot 70 may guide a saw blade to cut a posterior facet on the femur 2. A posterior chamfer cut slot 68 may guide a saw blade to cut a posterior chamfer facet on the femur 2. An anterior cut slot 64 may guide a saw blade to cut an anterior facet on the femur 2. An anterior chamfer cut slot 66 may guide a saw blade to cut an anterior chamfer facet on the femur 2. A trochlear recess cut slot 72 may guide a saw blade to cut the base of a trochlear recess on the femur 2. In addition, drill guide holes 74 may guide a drill bit to form post holes in the femur for receiving a fixation post of a femoral implant. Fixation holes 76 are positioned to receive additional pins, screws, or other fasteners to hold the cut guide 50 in place on the bone 2 while the saw cuts and drill holes are made.

In the illustrative guide 20 of FIG. 1, the holes 32, 34, 36 correspond to holes formed in cut guides 50 provided in a range of sizes. The central hole 32 in the orthopaedic guide 20 corresponds to the central hole 52 in the cut guide 50 and is common to all of the sizes of cut guides 50. The additional holes 54, 56 for receiving the datum pins 10 may vary in location by size of the cut guide 50. Therefore, the orthopaedic guide 20 includes multiple locations for the corresponding additional orthopaedic guide holes 34, 36. The additional orthopaedic guide holes 34, 36 may be labeled to identify the size of the cut guide 50 that is planned to be used. The datum pin 10 is then positioned using the correspondingly labeled orthopaedic guide hole 34, 36. Two pins 10 are sufficient to positively locate the cut guide 50.

The use of the orthopaedic guide 20 will now be described in conjunction with the exemplary femoral cut guide 50 surgical component in a procedure to replace the distal end of the femur 2 during knee joint replacement surgery. The surgeon may preoperatively determine the desired intraoperative size and location of the femoral implant. For example, X-ray images, CT data, MRI data, or other patient data may be digitized to form a computer model of the patient's anatomy and superimposed with a model of the available knee implants on a computer screen. The surgeon may then pick the appropriate size of implant and virtually maneuver it to a desired location in the computer model. This positioning information may then be used by the surgical navigation system to guide the surgeon to position the central common hole 32 in the orthopaedic guide 20 at the appropriate position to correctly position the chosen cut guide 50. For example, the surgeon may form the distal cut surface 4 in a conventional manner as is known in the art. The navigated orthopaedic guide 20 may then be positioned on the distal cut surface 4 and maneuvered about until the surgical navigation system indicates that the central hole 32 is in the required position. A datum pin 10 may then be inserted by drilling through the hole 32 into the femur 2 and pressing the datum pin 10 into the drilled hole 40. The orthopaedic guide 20 is thus fixed in a particular anterior-posterior (A/P) and medial-lateral (M/L) position and may now be rotated about the pin 10 in the central hole 32 until the surgical navigation system indicates that another hole 34, 36, corresponding to the planned implant size, is at the correct rotational position. A datum pin 10 may then be inserted by drilling through the appropriate hole 34, 36 into the femur 2 and pressing the datum pin 10 into the drilled hole 40. The orthopaedic guide 20 may now be removed by lifting it off of the datum pins 10. The appropriate femoral cut guide 50 may be positioned on the distal cut surface 4 of the femur 2 by sliding the cut guide 50 over the datum pins 10. The cut guide may be secured to the bone by inserting pins, screws, or other fasteners through one or more of the fixation holes 76 and into the femur 2. Saw blades and drills may be guided using the slots 64, 66, 68, 70, 72 and holes 74 in the cut guide 50 to prepare the femur 2 to receive a particular size of implant in a desired A/P, M/L, and rotational position.

Alternatively, the orthopaedic guide 20 may itself serve as a datum for guiding subsequent components. For example, the orthopaedic guide 20 may include a hole, slot, planar surface, and/or other feature for directly engaging and guiding a subsequent component relative to the surgical coordinate system. For example, the guide slots 64, 66, 68, 70, 72 and holes 74 of the cut guide 50 may be formed directly in the navigated guide 20. However, a navigated guide 20 with all of the features of the cut guide 50 may be more expensive and/or more delicate than the cut guide 50. Since the cut guides 50 are typically provided in a variety of sizes, it may be less costly and/or require less maintenance to provide a single separate navigated guide 20 for establishing a datum as described above. Furthermore, a separate navigated guide may be used to provide the benefits of surgical navigation technology while using existing non-navigated cut guides 50. This significantly reduces the cost of transition from a non-navigated to a navigated procedure by reducing the number of new instruments required.

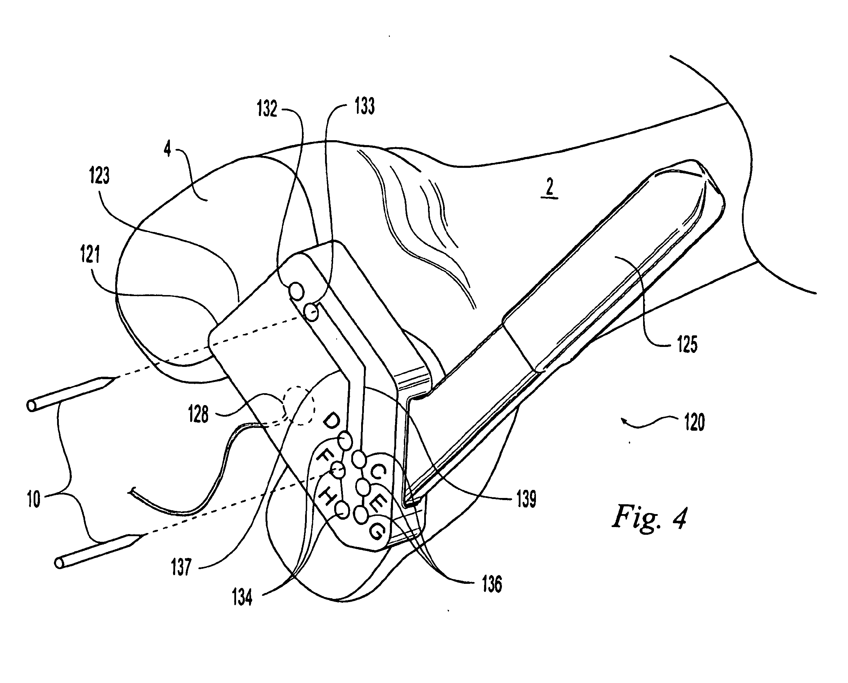

FIG. 4 illustrates an alternative arrangement for the navigated orthopaedic guide of FIG. 1. The orthopaedic guide 120 of FIG. 4 is approximately one-half the width of the orthopaedic guide 20 of FIG. 1. This smaller orthopaedic guide 120 is well suited for use in minimally invasive surgical procedures in which a reduced size incision is made. The guide 120 includes a body 121 and a tracking element in the form of an electromagnetic coil 128 to permit the surgical navigation system to track the position and orientation of the guide 120. A handle 125 extends from the guide 120 to facilitate insertion of guide into an incision. In a minimally invasive surgical procedure, it may be necessary to slip an edge 123 of the guide 120 under the margins of the incision such that the guide body 121 is largely covered by soft tissue. The handle 125 provides a gripping surface projecting from the incision. The guide body 121 includes a central hole 132 and first and second sets of additional datum guide holes 134, 136. The additional datum guide holes 134, 136 are labeled to indicate the corresponding cut guide 50 associated with each hole. In order to better accommodate the datum guide holes 134, 136 on a half size instrument, an alternate offset central hole 133 is provided. The alternate central hole 133 is associated with the second set of datum guide holes 136 so that the second set of datum guide holes 136 may be offset from and not overlap the first set of datum guide holes 134. A visual cue, such as etched lines 137, 139 may be provided to associate the corresponding central holes 132, 133 and additional datum guide holes 134, 136.

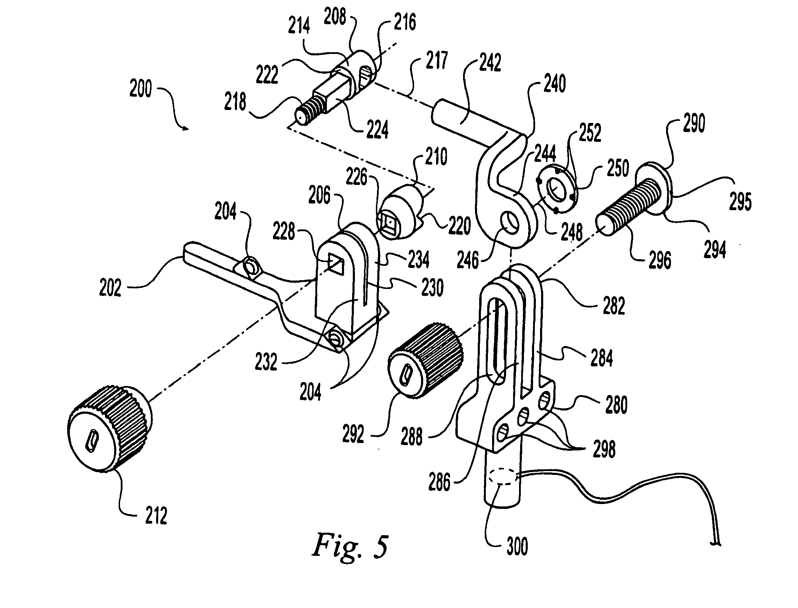

FIGS. 5-7 depict an illustrative alternative arrangement of the navigated orthopaedic guide of FIG. 1. further including an adjustment mechanism. The guide 200 includes a base member 202, a guide member 280 for establishing a datum, and a connecting link 240 connecting the base member 202 to the guide member 280. The base member 202 secures the guide 200 within the surgical navigation coordinate system. For example, the base member 202 may be secured to a bone adjacent the surgical site. The narrow elongated shape of the illustrative base member 202 permits it to fit into a narrow incision such as is used in a minimally invasive surgical technique. The illustrative base member 202 includes fixation holes 204 for receiving fixation members to secure the base member 202 to a bone. The fixation holes 204 may be angled to one side, as shown, to permit the fixation members to be inserted at an angle through a small incision and/or through a medially or laterally offset incision. The connecting link 240 permits adjustment of the guide member 280 relative to the base member 202 to permit the guide member 280 to be secured in a desired orientation relative to the bone. This adjustability is provided by adjustment mechanisms connecting the connecting link 240 to the base member 202 and the guide member 280.

The connecting link is connected to the base member 202 through a riser block 206 extending from the base member 202. A connecting link bolt 208 extends through a saddle washer 210, through the riser block 206, and into threaded engagement with a first locking knob 212. The connecting link bolt 208 includes a head 214 having a transverse bore 216. The connecting link 240 includes a cylindrical shaft 242 received by the transverse bore 216 for translation along and rotation about the bore 216 axis 217. As the first locking knob 212 is tightened onto the threads 218 of the connecting link bolt 208, the connecting link bolt 208 is drawn through the saddle washer 210 and riser block 206. The cylindrical shaft 242 of the connecting link 240 is drawn into abutment with a notch 220 in the saddle washer 210. Tightening of the first locking knob causes the saddle washer 210 to lock the connecting link 240 relative to the base member 202 and prevent translation and rotation of the connecting link relative to the base member 202. The connecting link bolt head 214 may be radially enlarged, for example to form a shoulder 222, so that the connecting link bolt 208 will not inadvertently pass through the saddle washer 210 and riser block 206 if the cylindrical shaft 242 is disengaged from the transverse bore 216. The connecting link bolt 208 may include a non-circular shaft portion 224 corresponding to non-circular bores 226, 228 in the saddle washer 210 and riser block 206 to prevent the connecting link bolt 208 from rotating relative to the base member 202. By constraining the connecting link bolt 208 against rotation, the only relative motion between the connecting link 240 and the base member 202 is translation along and rotation about the transverse bore axis 217. Furthermore, constraining the connecting link bolt 208 facilitates tightening the first locking knob 212.

The riser block 206 may include a slit 230 dividing the riser block into two cantilevered spaced apart portions 232, 234. These portions 232, 234 act as springs to provide a broader range of tension adjustment in the adjustment mechanism than would be possible without a spring. With the slit 230, the first locking knob 212 may be easily adjusted to a tension sufficient to hold the cylindrical shaft 242 in a desired position within the transverse bore 216 when acted on by the weight of the guide member 280 yet still allow a user to move the cylindrical shaft 242 in the transverse bore 216 with hand pressure. The first locking knob 212 may then be tightened to lock the cylindrical shaft 242 in the final desired position.

The connecting link 240 is connected to the guide member 280 through a tab 244 extending from the connecting link 240. The tab 244 includes a bore 246 having a bore axis 248 angled relative to the transverse bore axis 217. The angle between these bore axes 217, 248 permits a second degree of rotational adjustment of the guide member 280 relative to the base member 202. The guide member 280 includes a yoke 282 having first and second spaced apart arms 284, 286. Each arm 284, 286 includes an elongated slot 288 that permits a second degree of translation adjustment of the guide member 280 relative to the base member 202. The tab 244 is received between the arms 284, 286 in sliding and pivoting relationship. A guide member bolt 290 extends through one of the arms 284, through the bore 246 in the tab 244, through the other arm 286, and into threaded engagement with a second locking knob 292. This arrangement constrains the guide member 280 to rotation about the tab bore axis 248 and translation along the elongated slot 288. The guide member bolt 290 includes a radially enlarged head 294 that abuts one of the yoke arms 284 to prevent the bolt from pulling through the slot 288. As the second locking knob 292 is tightened onto the threads 296 of the guide member bolt 290, the yoke arms 284, 286 are flexed together to grip the tab 244 of the connecting link 240. The spring action of the arms 284, 286 permits a range of tab 244 gripping tension such that the second locking knob 292 may be easily adjusted to a tension sufficient to hold the tab 244 in a desired position within the yoke 282 when acted on by the weight of the guide member 280 yet still allow a user to rotate the tab 244 within the yoke 282 with hand pressure. The second locking knob 292 may then be tightened to lock the tab 244, and consequently the guide member 280, in the final desired position. One or more optional lock washers 250 may be provided between the tab 244 and yoke 282. The washer may include teeth 252 to increase the grip between the yoke 282 and tab 244. Furthermore, the guide member bolt head 294 may include a non-circular profile received in a corresponding recess (not shown) adjacent the slot 288 to prevent the bolt 290 from turning when the second locking knob 292 is tightened. For example, the bolt head 294 may have flat sides 295 that fit within a flat sided countersink (not shown) surrounding the slot 288.

The guide member 280 includes means for establishing a datum in the surgical navigation system coordinate system. In the illustrative orthopaedic guide of FIG. 5, the guide member 280 includes guide holes 298 for guiding pins to establish a datum. The guide member 280 includes a tracking element, such as an electromagnetic coil 300, to permit the surgical navigation system to track the position and orientation of the guide member 280.

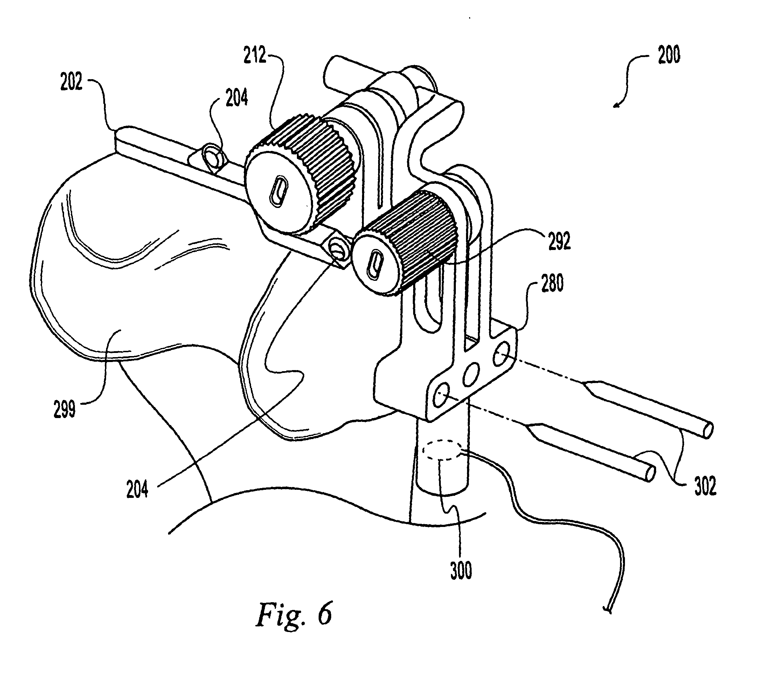

In use, the base member 202 is secured within the surgical navigation coordinate system by mounting it to an object known to the system. For example, the base member 202 may be mounted on a femur 299 as shown in FIG. 6. The narrow elongated shape of the illustrative base member 202 permits it to fit into a small incision. For example, the base member 202 may be inserted through a narrow medial or lateral incision adjacent to a knee joint. Furthermore, the fixation holes 204 may be angled, as shown, to permit fixation members to be inserted through such a medial or lateral incision. The first and second locking knobs 212, 292 are loosened to permit the guide member 280 to be moved relative to the base member 202. With the base member 202 positioned on the femur 299 as depicted in FIG. 6, the first locking knob 212 locks the medial-lateral position and the flexion angle of the guide member 280. The second locking knob 292 locks the varus-valgus position and resection depth of the guide member 280. The mechanism is manipulated until the surgical navigation system indicates that the guide member 280 is located in a desired position. The first and second locking knobs 212, 292 are then tightened to lock the guide member 280 in place relative to the base member 202. The guide member 280 may then be used to establish a datum for guiding a subsequent surgical component. For example, pins 302 may be inserted through guide holes 298 and into the femur 299. The navigated orthopaedic guide 200 may then be removed.

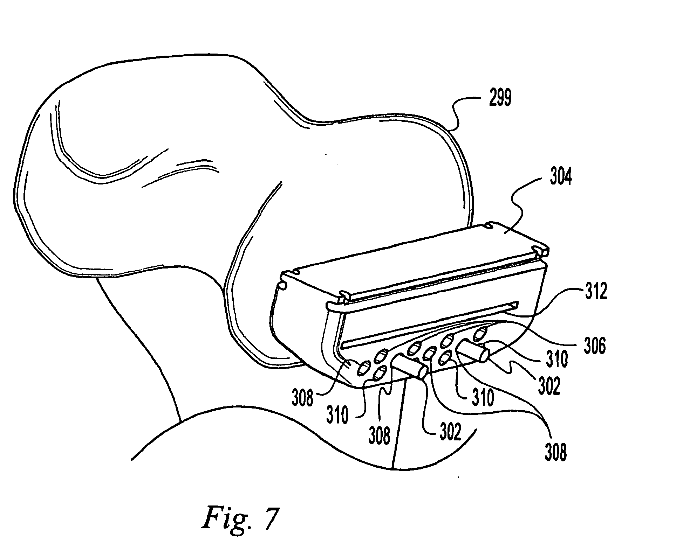

FIG. 7 illustrates a distal femoral cut block 304 mounted on the pins 302. The distal femoral cut block 304 includes holes 306, 308, 310 to receive the pins 302 and a cutter guide 312 for guiding a cutter to form a surface on the bone. The holes 306, 308, 310 may be provided as a plurality of rows of holes. Each row may provide a different level of resection. For example, one row of holes 308 may correspond to a predetermined nominal resection level. Additional rows 306, 310 may provide for cutting more or less bone should surgeon preference or the condition of the bone require it. By providing more holes in each row than the number of pins 302 used, the distal femoral cut block 304 may be adjusted anteriorly and posteriorly by lifting it off of the pins 302 and repositioning it on adjacent holes in the same row. With the cut block 304 positioned at the desired resection level and anterior-posterior position, additional fixation members may be inserted through some of the holes 306, 308, 310 to hold the cut block 304 in position while a cutter is guided to cut the bone 299.

The adjustable navigated orthopaedic guide 200 of FIGS. 5-7 has been shown configured to position a datum on the distal portion of a femur 299 to position a distal femoral cut guide 304. However, this adjustable guide may also be used to establish datums for other surgical components including cut guides such as a femoral finishing guide and/or a tibial cut guide. Also, as with the navigated orthopaedic guides of FIGS. 1-3 and 4, the guide of FIGS. 5-7 may itself serve as a datum to directly guide a subsequent surgical component.

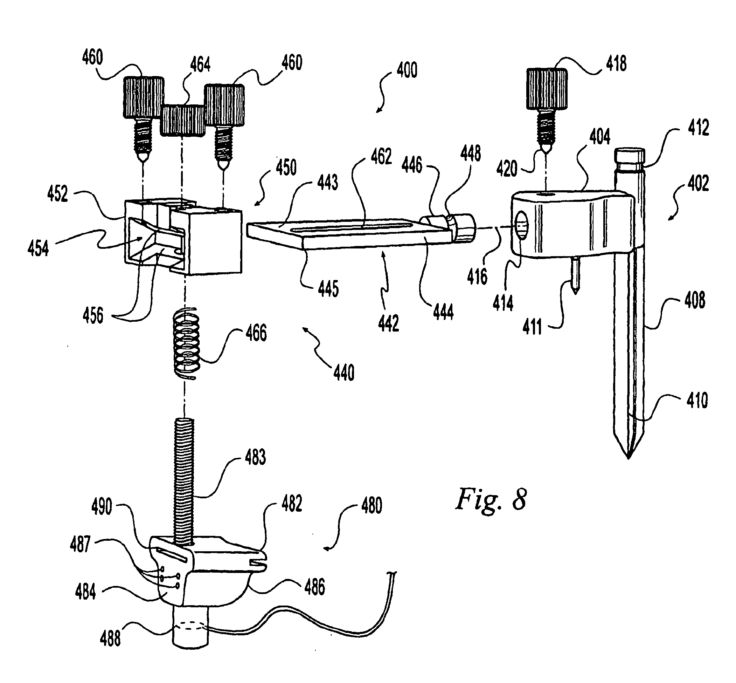

FIGS. 8-11 depict another illustrative alternative arrangement for the orthopaedic guide of FIG. 1 further including an adjustment mechanism. The guide 400 includes a base member 402, a guide member 480, and a connecting linkage 440 for adjustably connecting the base member 402 and the guide member 480. The base member 402 includes a receiver block 404 for receiving the connecting linkage 440 and an anchor portion 406 for securing the guide within the surgical navigation coordinate system. The illustrative anchor portion 406 includes a primary mounting post 408 that may be driven into a bone. The primary mounting post 408 may include fins 410 to resist rotation of the base member 402 relative to the bone. A supplemental mounting post 411 may also be included to resist rotation of the base member 402. The supplemental mounting post 411 may be spaced radially from the primary mounting post 408 to create a larger moment arm to resist rotation. The base member 402 may include means for gripping the base member 402 to remove it from the bone. The illustrative anchor portion 406 extends above the base member 402 and includes an annular groove 412 that may be engaged by a pin puller, slap hammer, and/or other suitable instrument to extract the base member 402.

The connecting linkage 440 permits adjustment of the guide member 480 relative to the base member 402 to permit the guide member 480 to be secured in a desired orientation relative to the bone. This adjustability is provided by adjustment mechanisms connecting the connecting linkage 440 to the base member 402 and the guide member 480.

The connecting linkage 440 is connected to the base member 402 by way of a rotating support 442. In the illustrative example, the rotating support 442 includes a plate-like body 444 having a top surface 443, a bottom surface 445, and a trunnion 446 projecting from one end. The trunnion 446 is received in a bore 414 formed in the receiver block 404 for rotation about the bore 414 axis 416. A set screw 418 is threaded into the receiver block 404 to lock the rotating support 442 in place. The trunnion 446 may include an annular groove 448 to receive the tip 420 of the set screw 418. With the set screw 418 loosely engaging the groove 448, the rotating support 442 may rotate about the bore axis 416 but it is prevented from translating along the bore axis 416. tightening the set screw 418 locks the rotating support 442 in its rotated position.

An adjustment screw housing 450 is supported at an opposite end of the rotating support 442. The housing 450 includes a body 452 with a transverse opening 454 defined by opposed fulcrums 456. The rotating support is 442 is received in the opening 454 with its top and bottom surfaces 443, 445 in close fitting relationship to the vertices 458 of the opposed fulcrums 456. The fulcrums 456 permit the housing 450 to rock relative to the rotating support 442. A pair of angle adjustment screws 460 is threaded into the adjustment screw housing 450 transverse to and in communication with the opening 454 such that the screws 460 may engage the top surface 433 of the rotating support 442. The screws 460 are positioned in the housing 450 so that they are on opposite sides of the fulcrum vertices 458. By loosening one of the angle adjustment screws 460 and tightening the other, the housing 450 will pivot on the fulcrum vertices 458 to allow adjustment of the angle of the housing 450 relative to the support 442.

The connecting linkage 440 is connected to the guide member 480 by means of a portion of the guide member 480 connecting to the housing 450. In the illustrative example, a threaded rod 482 projects from the guide member 480 and extends through the housing, through the vertices 458 of the opposed fulcrums 456, through an elongated slot 462 formed in the rotating support 442, and into threaded engagement with an adjustment nut 464. A spring 466 is interposed between the guide member 480 and housing 450 to bias them apart. Tightening the adjustment nut 464 draws the threaded rod 482 into the housing 450 and thereby moves the guide member 480 toward the housing 450 and compresses the spring 466. Loosening the adjustment nut 464 allows the guide member 480 to move away from the housing 450.

The guide member 480 includes means for establishing a datum in the surgical navigation system coordinate system. In the illustrative orthopaedic guide of FIG. 8, the guide member 480 includes a guide member body 483 having a front face 484 and a back face 486. Guide holes 487 for guiding pins to establish a datum extend from the front face 484 to the back face 486. The guide member 480 includes a tracking element in the form of an electromagnetic coil 488 to permit the surgical navigation system to track the position and orientation of the guide member 480.

The guide member 480 may optionally include a datum surface to directly guide a subsequent surgical component. The illustrative orthopaedic guide of FIGS. 8-11 includes a datum surface in the form of an elongated cutter guide slot 490 extending from the front face 484 to the back face 486 to directly guide a subsequent surgical component. If the optional direct guiding datum surface is provided, the holes 487 may receive fixation members to hold the guide member 480 in place while the guide member 480 directly guides a subsequent surgical component. Unlike femoral cut guides, which typically must be provided in a range of sizes, a single tibial cut guide is often able to be used to cut a wide variety of tibial sizes. Therefore, it may be advantageous to provide a single, direct guiding, orthopaedic guide configured for tibial use as shown. However, the orthopaedic guide of FIGS. 8-11 may also be used to establish datums for a separate surgical component such as a tibial cut guide, femoral cut guide, implant, and/or other surgical component. It may also advantageously be used to establish datums for existing tibial cut guides to provide the benefits of surgical navigation technology with existing non-navigated components.

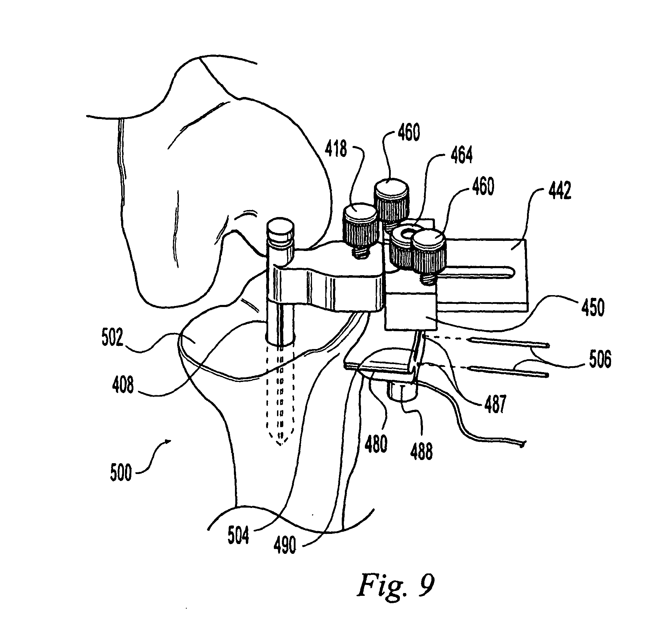

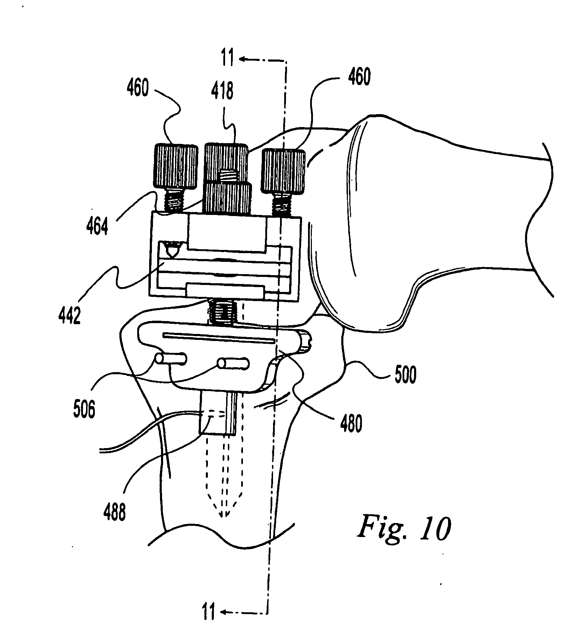

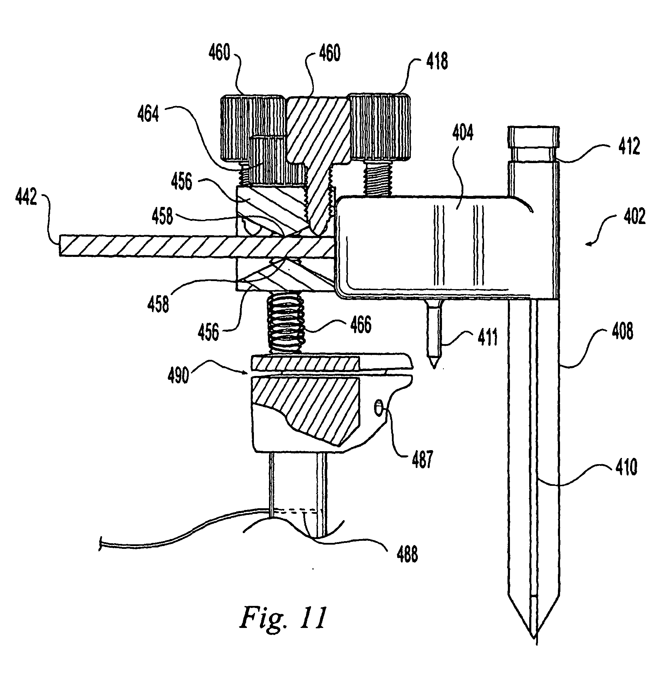

In use, the mounting post 408 is inserted into a bone to secure the guide 400 adjacent the bone, as shown in FIGS. 9 and 10. For use on a tibia 500, the mounting post 408 may be inserted through the proximal tibial surface 502 to position the guide member 480 adjacent the anterior tibial cortex 504. With both of the angle adjustment screws 460 loosened, the housing 450 and guide member 480 may be slid along the rotating support 442 to a desired position relative to the anterior tibial cortex 504. With the set screw 418 loosened, the rotating support 442, housing 450, and guide member 480 may be rotated to adjust the varus-valgus orientation of the guide member 480. By differentially tightening the angle adjustment screws 460, the housing 450 and guide member 480 may be angled about the fulcrum vertices 458 relative to the rotating support 442, as best seen in FIG. 11. this angle adjusts the posterior slope orientation of the guide member 480. Finally, by tightening or loosening the adjustment nut 464, the height of the guide member 480 may be varied to establish the resection depth position of the guide member 480. All of these adjustments may be made while the surgical navigation system is used to track the guide member 480. When the surgical navigation system indicates that the guide member 480 is in a desired position, the adjustment screws may be tightened to lock the position. The guide member 480 may now be used to establish a datum on the tibia 500, such as by inserting datum pins 506 through the holes 487 in the guide member 480 and into the anterior tibial cortex 504. The guide 400 may then be removed and the datum pins 506 may be engaged by a subsequent surgical component. For example, a cut block for guiding a cutter to resect the proximal tibial surface 502 may be engaged with the datum pins 506. Alternatively, the guide member 480 may directly establish a datum, such as with the guide slot 490, to guide a subsequent surgical component. For example, a cutter may be inserted in the guide slot 490 to guide the cutter to resect the proximal tibial surface 502.

The illustrative orthopaedic guide 400 of FIGS. 8-11 has been shown configured to directly guide a cutter to form a cut surface on the proximal tibia during a knee replacement surgical procedure. However, this orthopaedic guide 400 may also be used to directly guide or to establish datums for other surgical components and/or other surgical locations.

For example, the orthopaedic guide 400 may be used to directly guide, or establish datums to guide, instruments or implants into a desired position relative to the tibia or femur of the knee joint, the femur or pelvis of a hip joint, and/or other components and locations.

Although examples of a navigated orthopaedic guide and its use have been described and illustrated in detail, it is to be understood that the same is intended by way of illustration and example only and is not to be taken by way of limitation. The invention has been illustrated with orthopaedic guides setting pins or guiding cutters in specific locations related to knee replacement surgery. However, the orthopaedic guide may be configured to position other types of datums, for use with other types of surgical components, and at other locations within a patient's body. Accordingly, variations in and modifications to the orthopaedic guide and its use will be apparent to those of ordinary skill in the art, and the following claims are intended to cover all such modifications and equivalents.

## Claims

1. A navigated orthopaedic guide (20) for use with a surgical navigation system during an orthopaedic surgical procedure to establish a datum relative to a surgical site, the datum being able to be engaged by a subsequent surgical component to guide placement of the subsequent surgical component, the orthopaedic guide (20) comprising:
means for being tracked (28, 128) by the surgical navigation system to position the orthopaedic guide (20) at a desired position relative to the surgical site;
means for establishing a datum at a desired position relative to the surgical site such that the datum is able to be engaged by a subsequent surgical component to guide placement of the subsequent surgical component, the means for establishing a datum comprising a guide body (21, 121) including a plurality of holes (32, 34, 36, 7.32, 133, 134, 136) through the body (21, 121) for guiding the placement of one or more datums relative to the surgical site; wherein
the guide body (21, 121) comprises a body having a planar surface engageable with a distal cut surface (4) of a femur (2) and **characterised in that** the plurality of holes (32, 34, 36, 132, 133, 134, 136) includes one or more central holes (32, 132, 133) and a plurality of size specific holes (34, 36, 134, 136), each of the size specific holes (34, 36, 134, 136) corresponding to a different size of subsequent surgical component such that placement of a datum through the one or more central holes (32, 132, 133) and through one of the size specific holes (34, 36, 134, 136) results in a two feature datum engageable by a specific size of subsequent surgical component.

2. The navigated orthopaedic guide (20) of claim 1 **characterised in that** the means for establishing a datum comprises means for establishing one or more datums relative to the surgical site selected from the list consisting of pins, screws, bars, fins, rails, dovetails, planar surfaces, holes, slots, and/or notches.

3. The navigated orthopaedic guide (20) of claim 1 or claim 2 **characterised in that** the means for establishing a datum comprises means for establishing an intermediate datum separate from the guide (20) itself.

4. The navigated orthopaedic guide (20) of any one of the preceding claims **characterised in that** the means for establishing a datum comprises a body having a planar reference surface for engaging a distal femoral surface (4) of a femur (2) during knee replacement surgery, the body having a width approximately one-half the medial-lateral width of the distal femoral surface (4), the body having at least one hole (32, 34, 36, X 32, 133, 134, 136) for guiding a pin into engagement with the approximate center of the distal femoral surface (4) and at least one other hole (32, 34, 36, 132, 133, 134, 136) for guiding a pin into engagement with the distal femoral surface (4) to establish a datum comprising at least two pins engageable by a femoral finishing cut guide.

5. A surgical system for use during an orthopaedic surgical procedure at a surgical site of a patient's body, the system comprising the navigated orthopaedic guide of any one of the preceding claims and further comprising:
a surgical navigation system including means for tracking the position of an object during a surgical procedure; and
a surgical component including means for engaging the datum positioned by the orthopaedic guide (20) to locate the surgical component at a desired position relative to the surgical site.

6. The system of claim 5 **characterised in that** the means for tracking comprises multiple sensors to detect and triangulate the position of the orthopaedic guide (20).

7. The system of either claim 5 or claim 6 **characterised in that** the means for being tracked (28, 128) comprises an electromagnetic coil attached to the orthopaedic guide (20), the electromagnetic coil producing a signal detectable by the means for tracking.

8. The system of any one of claims 5 to 7 **characterised in that** the means for establishing a datum comprises a drill guide to guide a drill in forming a hole in a bone (2) at the surgical site.

9. The system of any one of claims 5 to 7 **characterised in that** the means for establishing a datum comprises at least one hole in the orthopaedic guide (20) to guide placement of a pin adj acent the surgical site.

10. The system of any one of claims 5 to 9 **characterised in that** the surgical component comprises an implant for replacing a portion of a bone (2).

11. The system of any one of claims 5 to 9 **characterised in that** the surgical component comprises a cut guide (50) to guide a cutter to cut a bone (2) to receive an implant.

12. The system of claim 11 **characterised in that** the cut guide (50) comprises a femoral finishing guide including guides for guiding a saw blade to shape the end of a femoral bone (2) to receive a femoral knee implant.

13. The system of claim 11 **characterised in that** the cut guide (50) comprises a distal femoral cut guide.

14. The system of claim 11 **characterised in that** the cut guide (50) comprises a proximal tibial cut guide.

15. The system of any one of claims 5 to 7 **characterised in that** the means for engaging the datum comprises at least one hole formed in the surgical component to receive the datum in the form of a pin.

16. The system of any one of claims 5 to 7 **characterised in that** the means for establishing a datum directly engages the subsequent surgical component.

## Patentansprüche

1. Navigierte orthopädische Führungseinrichtung (20) zur Verwendung mit einem chirurgischen Navigationssystem während einer orthopädischen chirurgischen Behandlung zum Positionieren eines Bezugselements relativ zu einer Operationsstelle, wobei eine später verwendete chirurgische Komponente mit dem Bezugselement in Eingriff gebracht werden kann, um die chirurgische Komponente zu führen und zu platzieren, wobei die orthopädische Führungseinrichtung (20) aufweist:
eine Einrichtung (28, 128), die durch das chirurgische Navigationssystem verfolgt werden kann, um die orthopädische Führungsvorrichtung (20) an einer gewünschten Position relativ zur Operationsstelle zu platzieren;
eine Einrichtung zum Errichten eines Bezugselements an einer gewünschten Position relativ zur Operationsstelle, so dass eine später verwendete chirurgische Komponente mit dem Bezugselement in Eingriff gebracht werden kann, um die Platzierung der chirurgischen Komponente zu führen, wobei die Einrichtung zum Errichten eines Bezugselements einen Führungskörper (21, 121) mit mehreren sich durch den Körper (21, 121) erstreckenden Löchern (32, 34, 36, 132, 133, 134, 136) zum Führen der Platzierung eines oder mehrerer Bezugselemente relativ zur Operationsstelle aufweist;
wobei der Führungskörper (21, 121) einen Körper mit einer ebenen Oberfläche aufweist, die dazu geeignet ist, mit einer distalen Schnittfläche (4) eines Oberschenkelknochens (2) in Eingriff zu kommen;
**dadurch gekennzeichnet, dass**
die mehreren Löcher (32, 34, 36, 132, 133, 134, 136) ein oder mehrere Mittellöcher (32, 132, 133) und mehrere größenspezifische Löcher (34, 36, 134, 136) aufweisen, wobei jedes der größenspezifischen Löcher (34, 36, 134, 136) einer anderen Größe einer später verwendeten chirurgischen Komponente entspricht, so dass durch Platzieren von Bezugselementen durch das eine oder die mehreren Mittellöcher (32, 132, 133) und durch eines der größenspezifischen Löcher (34, 36, 134, 136) ein Zwei-Merkmal-Bezugselement erhalten wird, mit denen eine später verwendete chirurgische Komponente mit einer spezifischen Größe in Eingriff bringbar ist.

2. Navigierte orthopädische Führungseinrichtung (20) nach Anspruch 1, **dadurch gekennzeichnet, dass** die Einrichtung zum Errichten eines Bezugselements eine Einrichtung zum Errichten eines oder mehrerer Bezugselemente relativ zur Operationsstelle aufweist, die aus Stiften, Schrauben, Stäben, Rippen, Schienen, Schwalbenschwanzelementen, ebenen Oberflächen, Löchern, Schlitzen und/oder Nuten ausgewählt werden.

3. Navigierte orthopädische Führungseinrichtung (20) nach Anspruch 1 oder 2, **dadurch gekennzeichnet, dass** die Einrichtung zum Errichten eines Bezugselements eine Einrichtung zum Errichten eines von der Führungseinrichtung (20) getrennten Zwischenbezugselements aufweist.

4. Navigierte orthopädische Führungseinrichtung (20) nach einem der vorangehenden Ansprüche, **dadurch gekennzeichnet, dass** die Einrichtung zum Errichten eines Bezugselements einen Körper mit einer ebenen Bezugsfläche aufweist, die dazu geeignet ist, während einer Knieersatzoperation mit einer distalen femoralen Fläche (4) eines Oberschenkelknochens (2) in Eingriff zu kommen, wobei der Körper eine Breite hat, die etwa der halben medial-lateralen Breite der distalen femoralen Fläche (4) gleicht, wobei der Körper mindestens ein Loch (32, 34, 36, 132, 133, 134, 136) zum Führen eines Stifts in Eingriff mit in etwa der Mitte der distalen femoralen Fläche (4) und mindestens ein anderes Loch (32, 34, 36, 132, 133, 134, 136) zum Führen eines Stifts in Eingriff mit der distalen femoralen Fläche (4) aufweist, um Bezugselemente zu erstellen, die mindestens zwei Stifte aufweisen, die mit einer Führungseinrichtung für einen femoralen Feinschnitt in Eingriff bringbar sind.

5. Chirurgisches System zur Verwendung während einer orthopädischen chirurgischen Behandlung an einer Operationsstelle des Körpers eines Patienten, wobei das System die navigierte orthopädische Führungseinrichtung nach einem der vorangehenden Ansprüche aufweist, ferner mit:
einem chirurgischen Navigationssystem mit einer Einrichtung zum Verfolgen der Position eines Objekts während einer chirurgischen Behandlung; und
einer chirurgischen Komponente mit einer Einrichtung, die dazu geeignet ist, mit dem durch die orthopädische Führungsvorrichtung (20) positionierten Bezugselement in Eingriff zu kommen, um die chirurgische Komponente an einer gewünschten Position relativ zur Operationsstelle anzuordnen.

6. System nach Anspruch 5, **dadurch gekennzeichnet, dass** die Verfolgungseinrichtung mehrere Sensoren zum Erfassen und Triangulieren der Position der orthopädischen Führungseinrichtung (20) aufweist.

7. System nach Anspruch 5 oder 6, **dadurch** gekennzeidchnet, dass die zu verfolgende Einrichtung (28, 128) eine an der orthopädischen Führungseinrichtung (20) angeordnete elektromagnetische Spule aufweist, wobei die elektromagnetische Spule ein durch die Verfolgungseinrichtung erfassbares Signal erzeugt.

8. System nach einem der Ansprüche 5 bis 7, **dadurch gekennzeichnet, dass** die Einrichtung zum Erstellen eines Bezugselementes eine Bohrerführungseinrichtung zum Führen eines Bohrers zum Ausbilden eines Lochs in einem Knochen (2) an der Operationsstelle aufweist.

9. System nach einem der Ansprüche 5 bis 7, **dadurch gekennzeichnet, dass** die Einrichtung zum Erstellen eines Bezugselementes mindestens eine Öffnung in der orthopädischen Führungseinrichtung (20) zum Führen der Platzierung eines Stifts in der Nähe der Operationsstelle aufweist.

10. System nach einem der Ansprüche 5 bis 9, **dadurch gekennzeichnet, dass** die chirurgische Komponente ein Implantat zum Ersetzen eines Teils eines Knochens (2) aufweist.

11. System nach einem der Ansprüche 5 bis 9, **dadurch gekennzeichnet, dass** die chirurgische Komponente eine Schnittführungseinrichtung (50) zum Führen einer Schneideinrichtung zum Zuschneiden eines Knochens (2) für die Aufnahme eines Implantats aufweist.

12. System nach Anspruch 11, **dadurch gekennzeichnet, dass** die Schnittführungseinrichtung (50) eine femorale Endbearbeitungsführungseinrichtung mit Führungen zum Führen eines Sägeblatts zum Formen des Endes eines Oberschenkelknochens (2) für die Aufnahme eines femoralen Knieimplantats aufweist.

13. System nach Anspruch 11, **dadurch gekennzeichnet, dass** die Schnittführungseinrichtung (50) eine distale femorale Schnittführungseinrichtung aufweist.

14. System nach Anspruch 11, **dadurch gekennzeichnet, dass** die Schnittführungseinrichtung (50) eine proximale tibiale Schnittführungseinrichtung aufweist.

15. System nach einem der Ansprüche 5 bis 7, **dadurch gekennzeichnet, dass** die Einrichtung, die dazu geeignet ist, mit dem Bezugselement in Eingriff zu kommen, mindestens ein in der chirurgischen Komponente ausgebildetes Loch zum Aufnehmen des Bezugselements in der Form eines Stifts aufweist.

16. System nach einem der Ansprüche 5 bis 7, **dadurch gekennzeichnet, dass** die Einrichtung zum Erstellen eines Bezugselementes mit der später verwendeten chirurgischen Komponente direkt in Eingriff kommt.

## Revendications

1. Guide orthopédique navigué (20) à utiliser avec un système de navigation chirurgicale pendant une procédure chirurgicale orthopédique pour établir un repère par rapport à un site chirurgical, le repère étant apte à être engagé par un composant chirurgical subséquent pour guider un placement du composant chirurgical subséquent, le guide orthopédique (20) comprenant:
un moyen devant être suivi (28, 128) par le système de navigation chirurgicale pour positionner le guide orthopédique (20) à une position désirée par rapport au site chirurgical;
un moyen pour établir un repère à une position désirée par rapport au site chirurgical de manière à ce que le repère soit apte à être engagé par un composant chirurgical subséquent pour guider un placement du composant chirurgical subséquent, le moyen pour établir un repère comprenant un corps guide (21, 121) incluant une pluralité de trous (32, 34, 36, 132, 133, 134, 136) à travers le corps (21, 121) pour guider le placement d'un ou davantage de repères par rapport au site chirurgical;
où
le corps guide (21, 121) comprend un corps ayant une surface planaire engageable avec une surface de coupe distale (4) d'un fémur (2) et **caractérisé en ce que** la pluralité de trous (32, 34, 36, 132, 133, 134, 136) inclut un ou davantage de trous centraux (32, 132, 133) et une pluralité de trous de tailles spécifiques (34, 36, 134, 136), chacun des trous de tailles spécifiques (34, 36, 134, 136) correspondant à une taille différente d'un composant chirurgical subséquent de manière à ce qu'un placement d'un repère à travers l'un ou les plusieurs trous centraux (32, 132, 133) et à travers l'un des trous de tailles spécifiques (34, 36, 134, 136) résulte en un repère à deux caractéristiques engageable par une taille spécifique d'un composant chirurgical subséquent.

2. Guide orthopédique navigué (20) de la revendication 1 **caractérisé en ce que** le moyen pour établir un repère comprend un moyen pour établir un ou davantage de repères par rapport au site chirurgical sélectionné à partir de la liste consistant en des broches, des vis, des barres, des ailettes, des rails, des queues d'aronde, des surfaces planaires, des trous, des fentes, et/ou des encoches.

3. Guide orthopédique navigué (20) de la revendication 1 ou la revendication 2 **caractérisé en ce que** le moyen pour établir un repère comprend un moyen pour établir un repère intermédiaire séparé du guide (20) lui-même.

4. Guide orthopédique navigué (20) de l'une quelconque des revendications précédentes **caractérisé en ce que** le moyen pour établir un repère comprend un corps ayant une surface de référence planaire pour engager une surface fémorale distale (4) d'un fémur (2) pendant une opération chirurgicale de remplacement de genou, le corps ayant une largeur d'approximativement la moitié de la largeur médiane-latérale de la surface fémorale distale (4), le corps ayant au moins un trou (32, 34, 36, 132, 133, 134, 136) pour guider une broche dans un engagement avec le centre approximatif de la surface fémorale distale (4) et au moins un autre trou (32, 34, 36, 132, 133, 134, 136) pour guider une broche dans un engagement avec la surface fémorale distale (4) pour établir un repère comprenant au moins deux broches engageables par un guide de coupe de finition fémorale.

5. Système chirurgical à utiliser pendant une procédure chirurgicale orthopédique à un site chirurgical d'un corps de patient, le système comprenant le guide orthopédique navigué de l'une quelconque des revendications précédentes et comprenant en plus:
un système de navigation chirurgicale incluant un moyen pour suivre la position d'un objet pendant une procédure chirurgicale; et
un composant chirurgical incluant un moyen pour engager le repère positionné par le guide orthopédique (20) pour localiser le composant chirurgical à une position désirée par rapport au site chirurgical.

6. Système de la revendication 5 **caractérisé en ce que** le moyen de suivi comprend plusieurs capteurs pour détecter et trianguler la position du guide orthopédique (20).

7. Système de la revendication 5 ou de la revendication 6 **caractérisé en ce que** le moyen devant être suivi (28, 128) comprend une bobine électromagnétique fixée au guide orthopédique (20), la bobine électromagnétique produisant un signal détectable par le moyen de suivi.

8. Système de l'une quelconque des revendications 5 à 7 **caractérisé en ce que** le moyen pour établir un repère comprend un guide de foret pour guider un foret pour former un trou dans un os (2) au site chirurgical.

9. Système de l'une quelconque des revendications 5 à 7 **caractérisé en ce que** le moyen pour établir un repère comprend au moins un trou dans le guide orthopédique (20) pour guider un placement d'une broche adjacente au site chirurgical.

10. Système de l'une quelconque des revendications 5 à 9 **caractérisé en ce que** le composant chirurgical comprend un implant pour remplacer une portion d'un os (2).

11. Système de l'une quelque des revendications 5 à 9 **caractérisé en ce que** le composant chirurgical comprend un guide de coupe (50) pour guider un instrument tranchant pour couper un os (2) devant recevoir un implant.

12. Système de la revendication 11 **caractérisé en ce que** le guide de coupe (50) comprend un guide de finition fémorale incluant des guides pour guider une lame de scie pour façonner l'extrémité d'un os fémoral (2) devant recevoir un implant fémoral de genou.

13. Système de la revendication 11 **caractérisé en ce que** le guide de coupe (50) comprend un guide de coupe fémorale distale.

14. Système de la revendication 11 **caractérisé en ce que** le guide de coupe (50) comprend un guide de coupe tibiale proximale.

15. Système de l'une quelconque des revendications 5 à 7 **caractérisé en ce que** le moyen pour engager le repère comprend au moins un trou formé dans le composant chirurgical pour recevoir le repère sous la forme d'une broche.

16. Système de l'une quelconque des revendications 5 à 7 **caractérisé en ce que** le moyen pour établir un repère engage directement le composant chirurgical subséquent.
